**Europäisches Patentamt**

**European Patent Office**

**Office européen des brevets**

(19)

(11) Veröffentlichungsnummer: **0 009 685**
**B1**

(12)

# EUROPÄISCHE PATENTSCHRIFT

(45) Veröffentlichungstag der Patentschrift:
**07.10.81**

(21) Anmeldenummer: **79103429.1**

(22) Anmeldetag: **13.09.79**

(51) Int. Cl.³: **C 07 D 213/12** // C07F15/06,
B01J31/12

(54) Verfahren zur Herstellung von 2-Vinylpyridin aus Acetylen und Acrylnitril.

(30) Priorität: **16.09.78 DE 2840460**

(43) Veröffentlichungstag der Anmeldung:
**16.04.80 Patentblatt 80/8**

(45) Bekanntmachung des Hinweises auf die Patenterteilung:
**07.10.81 Patentblatt 81/40**

(84) Benannte Vertragsstaaten:
**AT BE CH DE FR GB IT LU NL SE**

(56) Entgegenhaltungen:
**keine**

(73) Patentinhaber: **Studiengesellschaft Kohle mbH,
Kaiser-Wilhelm-Platz 1, D-4330 Mülheim/Ruhr (DE)**

(72) Erfinder: **Bönnemann, Helmut, Dr., Grashofstrasse 82,
Essen (DE)**
Erfinder: **Samson, Marc, Dr., Bismarckstrasse 28,
Mülheim/Ruhr (DE)**

(74) Vertreter: **von Kreisler, Alek, Deichmannhaus am
Hauptbahnhof, D-5000 Köln 1 (DE)**

## Verfahren zur Herstellung von 2-Vinylpyridin aus Acetylen und Acrylnitril

Die vorliegende Erfindung betrifft ein selektives Herstellungsverfahren von 2-Vinylpyridin aus Acetylen und Acrylnitril mit Hilfe löslicher Organo-Cobalt-Katalysatoren.

Der Erfindung liegt die Aufgabe zugrunde, ein Verfahren zu schaffen, das es gestattet, Acetylen und Acrylnitril selektiv und mit geringem Aufwand an Katalysator in 2-Vinylpyridin umzuwandeln.

Die bis heute vorgeschlagenen Verfahren sind hinsichtlich der Katalysatorausnutzung und Produktausbeuten wirtschaftlich unbefriedigend, wie eine Prüfung der in Tabelle 1 zusammengefassten Daten zeigt:

Tabelle 1

| Literatur | Katalysator | Temp. °C | Nitril-Konzentration Mol/l | Verweilzeit Min | Pyridin-Ausbeute, bez. auf Acrylnitrilumsatz | Produktbildungsschritte pro Co-Atom |
|---|---|---|---|---|---|---|
| Dos 26 153 09 C.P. Hardt, Lonza AG Beispiel 3 | Cobaltocen | 120 | 4,57 | 60 | 41,6 | 38 |
| H. Yamazaki, J. Wakatsuki Synthesis 1976, 26 pag. 28 | Cobaltocen | 110 | 5,05 | 420 | 31,0% | 48,0 |
| US-PS 4,006,149 Studiengs. Kohle mb.H. Beispiel 40 | Cyclopentadienyl-Co-Cyclooctadien | 100 | 2,144 | 240 | 75,0% | 63,0 |

Yamasaki et al erhalten eine Ausbeute von 31% an 2-Vinylpyridin mit Cobaltocen als Katalysator. Die Synthese wird bei 110 °C bei einer Reaktionsdauer von 7 Stunden und einer Konzentration an Acrylnitril von 5,05 Mol/l durchgeführt. Die Reaktion war mit Absicht bei einer deutlich niedrigeren Temperatur, nämlich 110 °C, vergleichsweise zur Herstellung anderer Pyridinderivate ausgeführt worden, um den Verlust an Vinylpyridin durch Polymerisation herabzudrücken. Eine Verringerung der Reaktionsdauer auf 60 Minuten führt nach C.P. Hardt unter Verwendung des gleichen Katalysators, leicht erhöhter Temperatur (120 °C) und leicht ermässigter Nitrilkonzentration 4,57 Mol/l) nicht zum Erfolg. Die Ausbeute bleibt unter 45%, d.h. für eine wirtschaftliche Verwertung unbefriedigend niedrig.

In der US-PS 4 006 149 sind Kobalt-I-Komplexverbindungen als Katalysatoren unter anderem für die Herstellung von Vinylpyridinen genannt, die vergleichsweise zu dem Katalysator, Kobaltocen, der vorgenannten Offenbarungen wesentlich wirkungsvoller sind. Bei 100 °C und einer Verweilzeit von 4 Stunden werden bei einer Nitrilkonzentration von 2,14 Mol/l immerhin 75% Ausbeute erzielt. Bei der Wahl der vorgenannten Parameter — Absenkung der Temperatur und der Nitrilkonzentration, mittlerer Reaktionsdauer — war nicht zu erwarten, dass unter Verwendung von z.B. Cobaltocen als Katalysator eine wesentliche Verbesserung des Syntheseergebnisses erzielt werden konnte.

Überraschenderweise wurde nun entgegen jeder Erfahrung gefunden, dass die Ausbeute an polymerisationsempfindlichem 2-Vinylpyridin drastisch erhöht werden kann, wenn man die Temperatur auf 150 °C und darüber erhöht, die Verweilzeit unter 50 Minuten herabsetzt und die Nitrilkonzentration unter 2 Mol/l wählt. Geeignete Katalysatoren für diese selektive Synthese von 2-Vinylpyridin sind dann generell Cyclopentadienyl-Kobalt-Verbindungen bzw. π-Allyl-Kobalt-Verbindungen.

Für die Wahl der Temperatur ist der Bereich um 150–160 °C vorteilhaft, ein Temperaturbereich von 140–180 °C aber auch anwendbar.

Die Reaktionsdauer liegt bei etwa 5 Minuten bis zu höchstens 50 Minuten, kann aber auch im Bereich unterhalb 5 Minuten bis zu einer Grössenordnung von Sekunden liegen, wenn entsprechende Reaktionsapparaturen, wie z.B. Strömungsrohre, verwendet werden. Vorteilhaft ist im allgemeinen eine Reaktionsdauer von 15 bis 30 Minuten.

Die Konzentration an Acrylnitril liegt in einem Bereich von etwa 0,1 Mol/l bis zu 2 Mol/l oder auch darüber. Vorzugsweise liegt die Konzentration an Acrylnitril um oder über 1 Mol/l.

Als Lösungsmittel sind solche geeignet, die gegenüber den Reaktionspartnern im angegebenen Temperaturbereich inert sind. Solche sind beispielsweise aliphatische Lösungsmittel und insbesondere aromatische Lösungsmittel wie Benzol oder Toluol.

Zweckmässig führt man die erfindungsgemässe Umsetzung in einem Druckgefäss mit guter Durchmischungseinrichtung durch, damit während der Umsetzung eine Sättigung der Lösung mit Acetylen gewährleistet wird. Der Acetylendruck liegt dabei zwischen 5 und 20 Atm., vorzugsweise zwischen 8 und 17 Atm.

Geeignete Katalysatoren für das erfindungsgemässe Verfahren sind temperaturbeständige Kobaltverbindungen, die mindestens ein Cyclopentadienyl am Kobalt gebunden enthalten, wie beispielsweise Bis-Cyclopentadienylcobalt (Cobaltocen), Cyclopentadienyl-Kobalt-cyclooctadien, Cyclopentadienyl-Kobalt-dicarbonyl aber auch $\pi$-Allyl-Kobalt-Verbindungen.

Das Verfahren kann diskontinuierlich aber alternativ auch kontinuierlich durchgeführt werden, so entweder in einer Rührkesselkaskade oder im Strömungsrohr, wobei eine rasche Abkühlung des Auslaufstromes sichergestellt sein sollte. Der Katalysator wird zweckmässigerweise in Form vorgefertigter Kobaltverbindungen eingesetzt, er kann aber auch in der Reaktionsmischung in situ hergestellt werden.

Das erfindungsgemäss hergestellte 2-Vinylpyridin findet als Komponente von terpolymeren Haftvermittlern in der Reifenindustrie Verwendung und dient ferner als Comonomer bei der Acrylfaser-Produktion.

Beispiel 1

0,12 g (0,52 mMol) Cyclopentadienyl-Kobalt-cycloocta-(1,5)-dien löst man in 241,6 ml Toluol auf und versetzt mit 14,05 g (264,8 mMol) Acrylnitril, wobei 259,1 ml einer 1,02 molaren Nitrillösung einschliesslich Katalysator resultieren. Diese wird bei 20 °C in einen 500 ml Edelstahlautoklaven mit magnetischer Rühreinrichtung eingesaugt. Man sättigt die Lösung bei 7 Bar mit Acetylen und heizt innerhalb 15 Minuten auf 150 °C auf, wobei der Druck durch Zupressen von Acetylen auf 17–18 erhöht wird. Verbrauchtes Acetylen ergänzt man laufend. Nach 30 Minuten Verweilzeit bei 150 °C bringt man das Druckgefäss binnen 10 Minuten durch äussere Kühlung auf 30 °C.

233 g Rohprodukt werden aus dem Autoklaven ausgefüllt und die flüchtigen Bestandteile bei 0,2 Torr abkondensiert, wobei 1,90 g Rückstand hinterbleiben. Das Kondensat, 230,0 g enthielt laut gaschromatographischer Analyse 8,13 g Acrylnitril und 10,88 g 2-Vinylpyridin.

Ausbeute, bez. auf umgesetztes

| | |
|---|---|
| Acrylnitril: | 93% 2-Vinylpyridin |
| Katalysatorausnutzung: | 200 Mol Pyridin/g At Kobalt |

Beispiel 2

Man verfährt wie in Beispiel 1 beschrieben, verwendet jedoch Cobaltocen als Katalysator.

Eingesetzt:

| | | |
|---|---|---|
| 0,2 g | (1,06 mMol) | Cobaltocen |
| 13,5 g | (246 mMol) | Acrylnitril } 0,95 molare Lösung |
| 209,9 g | (242 ml) | Toluol |

Bedingungen:

| | |
|---|---|
| Reaktionstemperatur: | 148 °C |
| Verweilzeit bei 148 °C: | 50 Minuten |
| Reaktionsaustrag: | 231,20 g |
| Kondensat (0,2 Torr): | 227,70 g |
| Rückstand: | 3,05 g |

Ergebnis:
(lt. gaschromatographischer Analyse)
6,25 g   nicht umgesetztes Acrylnitril
10,44 g   2-Vinylpyridin

Ausbeute, bez. auf umgesetztes

| | |
|---|---|
| Acrylnitril: | 78% 2-Vinylpyridin |
| Katalysatorausnutzung: | 94 Mol Pyridin/g At Kobalt |

Beispiel 3

Man verfährt wie in Beispiel 1 beschrieben, verwendet jedoch π-Cyclopentadienyl-Dicarbonyl-Kobalt als Katalysator.

Eingesetzt:

| | | |
|---|---|---|
| 0,135 g | (0,86 mMol) | $\pi$-C$_5$H$_5$Co(CO)$_2$ |
| 20,7 g | (390,6 mMol) | Acrylnitril |
| 203,5 g | (234 ml) | Toluol |

} 1,50 molare Lösung

Bedingungen:

| | |
|---|---|
| Reaktionstemperatur: | 155 °C |
| Verweilzeit bei 155 °C: | 30 Minuten |
| Reaktionsaustrag: | 229,5 g |
| Kondensat (0,2 Torr): | 228,3 g |
| Rückstand: | 0,7 g |

Ergebnis:
(lt. gaschromatographischer Analyse)
15,12 g   nicht umgesetztes Acrylnitril
 8,89 g   2-Vinylpyridin

Ausbeute, bez. auf umgesetztes
Acrylnitril:                          80,4 g% 2-Vinylpyridin
Katalysatorausnutzung:    98 Mol Pyridin/g At Kobalt

Beispiel 4

Man verfährt wie in Beispiel 1 beschrieben, verwendet jedoch π-Cyclopentadienyl-(1-exo-trichloromethylcyclopentadien)-Kobalt als Katalysator.

Eingesetzt:

| | | |
|---|---|---|
| 0,245 g | (0,797 mMol) | $\pi$-C$_5$H$_5$CoC$_5$H$_5$CCl$_3$ |
| 13,65 g | (257,6 mMol) | Acrylnitril |
| 206,4 g | (237 ml) | Toluol |

} 1,02 molare Lösung

Bedingungen:

| | |
|---|---|
| Reaktionstemperatur: | 150 °C |
| Verweilzeit bei 150 °C: | 30 Minuten |
| Reaktionsaustrag: | 223,6 g |
| Kondensat (0,2 Torr): | 222,8 g |
| Rückstand: | 0,6 g |

Ergebnis:
(lt. gaschromatographischer Analyse)
9,73 g   nicht umgesetztes Acrylnitril
6,37 g   2-Vinylpyridin

Ausbeute, bez. auf umgesetztes
Acrylnitril:                          82% 2-Vinylpyridin
Katalysatorausnutzung:    76 Mol Pyridin/g At Kobalt

Beispiel 5

Man verfährt wie in Beispiel 1 beschrieben, verwendet jedoch π-Cyclopentadienyl-(1-exo-cyanomethylencyclopentadien)-Kobalt als Katalysator.

---

Eingesetzt:

| | | |
|---|---|---|
| 0,206 g | (0,897 mMol) | π-$C_5H_5CoC_5H_5CH_2CN$ |
| 16,25 g | (306,6 mMol) | Acrylnitril |
| 209,6 g | (241 ml) | Toluol |

} 1,17 molare Lösung

Bedingungen:

| | |
|---|---|
| Reaktionstemperatur: | 155 °C |
| Verweilzeit bei 155 °C: | 30 Minuten |
| Reaktionsaustrag: | 236,75 g |
| Kondensat (0,2 Torr): | 232,6 g |
| Rückstand: | 4,0 g |

Ergebnis:
(lt. gaschromatographischer Analyse)
 7,27 g   nicht umgesetztes Acrylnitril
13,74 g   2-Vinylpyridin

Ausbeute, bez. auf umgesetztes

| | |
|---|---|
| Acrylnitril: | 77% 2-Vinylpyridin |
| Katalysatorausnutzung: | 146 Mol Pyridin/g At Kobalt |

---

Beispiel 6

Man löst 0,2 g (0,9 mMol) Methylheptadienyl-Butadien-Kobalt bei − 30 °C in 233 ml Toluol und versetzt mit 0,05 g (0,94 mMol) monomerem Cyclopentadien. Man erwärmt auf 20 °C, gibt 19,2 g (362,3 mMol) Acrylnitril zu (es resultiert eine 1,41 molare Lösung von Acrylnitril in Toluol) und verfährt im übrigen analog Beispiel 1.

---

Bedingungen:

| | |
|---|---|
| Reaktionstemperatur: | 155 °C |
| Verweilzeit bei 155 °C: | 30 Minuten |
| Reaktionsaustrag: | 227,9 g |
| Kondensat (0,2 Torr): | 225,8 g |
| Rückstand: | 1,85 g |

Ergebnis:
(lt. gaschromatographischer Analyse)
12,69 g   nicht umgesetztes Acrylnitril
 9,74 g   2-Vinylpyridin

Ausbeute, bez. auf umgesetztes

| | |
|---|---|
| Acrylnitril: | 75,3% 2-Vinylpyridin |
| Katalysatorausnutzung: | 103 Mol Pyridin/g At Kobalt |

---

Beispiel 7

Man verfährt wie in Beispiel 1 beschrieben, verwendet jedoch π-Indenyl-Cyclooctadien-1,5-Kobalt als Katalysator.

---

Eingesetzt:

| | | |
|---|---|---|
| 0,1492 g | (0,529 mMol) | Indenyl-Kobalt-COD |
| 26,20 g | (494,3 mMol) | Acrylnitril (1,65 molar) |
| 231,25 g | | Toluol |

Bedingungen:
| | |
|---|---|
| Reaktionstemperatur: | 140 °C |
| Verweilzeit bei 140 °C: | 20 Minuten |
| Reaktionsaustrag: | 271,15 g |
| Kondensat:(0,2 Torr): | 268,3 g |
| Rückstand: | 2,6 g |

Ergebnis:
(lt. gaschromatographischer Analyse)
16,79 g   nicht umgesetztes Acrylnitril
16,44 g   2-Vinylpyridin

Ausbeute, bez. auf umgesetztes
| | |
|---|---|
| Acrylnitril: | 88% 2-Vinylpyridin |
| Katalysatorausnutzung: | 294 Mol Pyridin/g At Kobalt |

**Beispiel 8**

101,4 mg (0,3595 mMol) π-Indenyl-Kobalt-COD löst man in 151,8 g Toluol auf und versetzt mit 19,0 g (358,5 mMol) Acrylnitril, wobei 200 ml einer 1,79 molaren Nitrillösung einschliesslich Katalysator resultieren. Diese Lösung wird bei 20 °C in einem 500-ml-Edelstahlautoklaven eingesaugt. Man sättigt die Lösung mit 7 bar Acetylen. Innerhalb von 12 Minuten heizt man auf 145 °C auf, wobei der Druck durch Zupressen von Acetylen auf 18,5 bar erhöht und konstant gehalten wird. Nach 17 Minuten Reaktionszeit dosiert man 105,5 mg (0,374 mMol) Indenyl-Kobalt-COD, 29,9 g Toluol gelöst, innerhalb von 6 Minuten zu. Nach 18 Minuten Reaktionszeit bei 145 °C kühlt man das Druckgefäss binnen 10 Minuten auf 25 °C ab.

| | |
|---|---|
| Reaktionszeit: | 41 Minuten |
| Reaktionstemperatur: | 145 °C |
| Reaktionsaustrag: | 210,7 g |
| Kondensat (0,2 Torr): | 206,6 g |
| Rückstand: | 2,9 g |

Ergebnis:
(lt. gaschromatographischer Analyse)
9,31 g   nicht umgesetztes Acrylnitril
15,4 g   2-Vinylpyridin

Ausbeute, bez. auf umgesetztes
| | |
|---|---|
| Acrylnitril: | 79% 2-Vinylpyridin |
| Katalysatorausnutzung: | 197 Mol Pyridin/g At Kobalt |

**Beispiel 9**

Darstellung von 2-Vinylpyridin mit Trimethylsilylcyclopentadienyl-(cycloocta- 1,5-dien)-Kobalt als Katalysator

Eingesetzt:
| | | |
|---|---|---|
| 0,222 g | (0,730 mMol) | Me₃SiCpCoCOD |
| 15,55 g | (293,40 mMol) | Acrylnitril (1,13 molar) |
| 208,65 g | Toluol | |

Bedingungen:
| | |
|---|---|
| Reaktionstemperatur: | 150 °C |
| Reaktionszeit: | 30 Min. |
| Reaktionsaustrag: | 233,55 g |
| Kondensat: (0,2 Torr) | 231 g |
| Rückstand: | 2,0 g |

Ergebnis:
(lt. gaschromatographischer Analyse)
9,51 g   Acrylnitril (nicht umgesetzt)
10,94 g   2-Vinylpyridin

Ausbeute, bez. auf umgesetztes
Acrylnitril: 91% 2-Vinylpyridin
Katalysatorausnutzung: 143 Mol Pyridin/g-At Kobalt

Beispiel 10
Darstellung von 2-Vinylpyridin mit π-Cyclopentadienyl-(α,α'-Bipyridyl)-Kobalt als Katalysator

Eingesetzt:
| 0,150 g | (0,536 mMol) | CpCobipy |
| 16,70 g | (315,1 mMol) | Acrylnitril (1,21 molar) |
| 209,25 g | Toluol | |

Bedingungen:
Reaktionstemperatur: 145 °C
Reaktionszeit: 15 Min.
Reaktionsaustrag: 233,50 g
Kondensat: (0,2 Torr) 229,80 g
Rückstand: 1,5 g

Ergebnis:
(lt. gaschromatographischer Analyse)
10,55 g Acrylnitril (nicht umgesetzt)
9,82 g 2-Vinylpyridin

Ausbeute, bez. auf umgesetztes
Acrylnitril: 81% 2-Vinylpyridin
Katalysatorausnutzung: 174 Mol Pyridin/g-At Kobalt

Darstellung von π-Indenyl-(cycloocta-1,5-dien)-Kobalt

$C_8H_{13}CoC_4H_6$ + $C_9H_8$ ⟶ $C_9H_9Co(dien)$
(222) (116)
Natta-Komplex Inden
$C_9H_9Co(dien)$ + COD-1,5 ⟶ $C_9H_9CoCOD$
(108) (284)
Ind Co COD

Man versetzt 3,8 g (17,1 mMol) Natta-Komplex, gelöst in 100 ml Pentan, mit 1,98 g (17,1 mMol) Inden bei −30 °C. Danach wird die Reaktionslösung langsam auf RT gebracht, und man lässt noch 20 h nachreagieren. Anschliessend wird ca. 5 ml COD-1,5 zugegeben. Die klare rote Lösung wird ca. 24 h zum Rückfluss erhitzt. Nach Abkühlen wird evtl. entstandener Niederschlag abfiltriert und die Lösung auf ca. 30 ml eingeengt und langsam bis auf −50 °C gekühlt. Es entstehen braunrote Kristalle. Die überstehende Mutterlauge drückt man ab und trocknet die Kristalle (rotbraungefärbte Nadeln) 1 h bei RT im Hochvakuum (10⁻⁴ Torr).

Ausbeute: 2,4 g (50% d.Th.)
Smp.: 98 °C
Elementaranalyse:
gefunden C: 73.20%; H: 6.86%; Co: 19.29%
berechnet C: 72.34%; H: 6.78%; Co: 20.88%

IR-Analyse:
1450; 1325; 1315; 1200; 1030; 960; 905; 840; 790; 735; 725 cm⁻¹

¹H–NMR (d₈-Toluol, 80 MHz):

δH₁: 7.14 (s)
δH₂: 5.78 (t, J=2.4 Hz)
δH₃: 3.89 (d, J=2.4 Hz)
δH₄: 3.40 (m)
δH₅: 2.09 (m)
δH₆: 1.40 (m)

¹³C-NMR (d₈-Toluol):

$\delta C_1$: 67.38 (d, $J_{C,H}$=152 Hz)
$\delta C_2$: 31.35 (t, $J_{C,H}$=127 Hz)
$\delta C_3$: 88.51 (d, $J_{C,H}$=174.6 Hz)
$\delta C_4$: 75.80 (d, $J_{C,H}$=174.4 Hz)
$\delta C_5$: 105.48 (s)
$\delta C_6$: 124.27 (d, $J_{C,H}$=159 Hz)
$\delta C_7$: 122.98 (d, $J_{C,H}$=162 Hz)

Massenspektrum:
m/e: 282 (M$^+$·, 100%); 252 (42%); 174 (80%); 164 (25%); 138 (28%); 124 (30%); 115 (45%); 59 (15%)

Lit.:
«π-Indenyl-(cyclooctatrien)-Cobalt», A. Greco, M. Green, F. Stone, J. Chem. Soc. (A), 286 (1971)

Darstellung von π-Trimethylsilylcyclopentadienyl(cycloocta-1,5-dien)-Kobalt

| | CpH | + n-BuLi | — | LiCp+n-Butan |
|---|---|---|---|---|
| | (66) | | | |
| (1) | LiCp | + Me$_3$SiCl | — | Me$_3$SiCp+LiCl |
| | (72) | (108.5) | | (138) |
| (2) | 2 Me$_3$SiCp | + Co$_2$(CO)$_8$ | — | 2 Me$_3$SiCpCo(CO)$_2$+4 CO+H$_2$ |
| | (138) | (342) | | (252) |
| (3) | Me$_3$SiCpCo(CO)$_2$ | + COD-1,5 | — | Me$_3$SiCpCoCOD+2 CO |
| | (252) | (108) | | (304) |

(1) 33 g (0,5 Mol) Cyclopentadien werden bei Raumtemperatur vorgelegt und mit 200 ml Benzol und 100 ml Tetrahydrofuran verdünnt. Innerhalb von 2 h werden 213 ml einer 15proz. Lösung Butyllithium in Hexan zugetropft. Anschliessend werden 64,5 (0,5 Mol) Trimethylsilylchlorid, gelöst in 100 ml Benzol, zugetropft und die Reaktionsmischung 15 h unter Rückfluss gekocht. Nach Abkühlen wird der entstandene weisse Niederschlag (LiCl) abfiltriert und mit Benzol nachgewaschen. Das Filtrat wird eingeengt und anschliessend destilliert.
Sdp.: 60–65 °C/Wasserstrahlpumpe ca. 10 Torr
Ausbeute: 30 g (46.5% d,Th.)
(2) Zu einer Lösung von 3,42 g (10 mMol) Co$_2$(CO)$_8$ in 20 ml Äther werden ca. 10 ml Trimethylsilylcyclopentadien gegeben. Die Mischung wird 1 h bis zum Rückfluss erhitzt. Nach Filtration wird das Lösungsmittel abgezogen und der Rückstand destilliert.
Sdp.: 50–52 °C/0,5 Torr
Ausbeute: 4,3 g 85% d. Th.)
(3) Eine Lösung von 3,90 g (14,8 mMol) Me$_3$SiCp-Co(CO)$_2$ in 10 ml COD-1,5 wird 12 h bis zum Rückfluss erhitzt. Nach Abkühlen wird die Lösung eingeengt, und man erhält als Rückstand eine hellbraune kristalline Masse. Umkristallisation aus Pentan bei −40 °C liefert das reine Produkt.
Ausbeute: 3,6 g (80% d. Th.)
Smp.: 64 °C

Elementaranalyse:
gefunden C: 64.54%; H: 7.98%; Co: 18.53%; Si: 8.82%
berechnet C: 63.16%; H: 8.55%; Co: 19.41%; Si: 9.04%
IR-Analyse:
1440; 1360; 1315; 1240; 1155; 1055; 1040; 900; 875; 850; 820 (s); 800; 740; 680; 620 cm$^{-1}$.

$^1$H-NMR (d$_8$-Toluol, 60 MHz):

$\delta H_1$: 0.0 (s), innerer Standard
$\delta H_2$: 3.0 (t, J=2 Hz)
$\delta H_3$: 4.38 (t, J=2 Hz)
$\delta H_4$: 1.22 (m)
$\delta H_5$: 2.0 (m)
$\delta H_6$: 3.0 (m)

Massenspektrum:
m/e: 304 (M$^+$·, 45%); 229 (100%); 196 (40%); 181 (18%); 137 (15%); 124 (15%); 107 (20%); 93 (10%); 73 (18%)

Lit.:
1. Darstellung Me$_3$SiCpH: K.C.Frisch, J. Am. Chem. Soc. 75, 6050 (1953).
2. Darstellung Me$_3$SiCpCo(CO)$_2$: E.W. Abel, S. Moorhause, J. Organomet. Chem. 28, 211–215 (1971).
3. Allgemeine Lit. SiCp-Met.-Verbindungen: «Advances in Organometallic Chemistry», Vol. 15 (1977), I. Haidue, V. Popa, S. 113 (Academic Press).

Darstellung von π-Cyclopentadienyl-α,α'-Bipy-ridyl-Kobalt

| | | | | |
|---|---|---|---|---|
| (1) | CpCo(CO)$_2$ (180) | + α,α'-Bipyridyl (156) | — | CpCobipy+2 CO (280) |
| (2) | CpCo(CH$_2$=CH$_2$)$_2$ (180) | + α,α'-Bipyridyl (156) | — | CpCobipy+2 CH$_2$=CH$_2$ (280) |

(1) Zu einer Lösung von 8,15 g (45,3 mMol) Cyclopentadienylcobaltdicarbonyl in 100 ml Xylol wird bei RT eine Lösung von 7,06 (45,3 mMol) α,α'-Bipyridyl in 50 ml Xylol zugetropft. Anschliessend wird bis zum Rückfluss erhitzt. Der Fortschritt der Reaktion ist daran zu erkennen, dass abnehmende Mengen CpCo(CO)$_2$ mit dem Lösungsmittel mitgerissen werden bis schliesslich der Rückfluss farblos kondensiert; ca. 12 h Reaktionszeit bei 160 °C. Die Farbe ändert sich während der Reaktion von tiefrot nach tiefviolett. Abkühlen und Einengen des Lösungsmittels liefert einen schwarzvioletten Niederschlag, der aus Pentan bei −50 °C umkristallisiert wird.
Ausbeute: 10,7 g (84% d.Th.)

Elementaranalyse:
gefunden   C: 64.01%;   H: 5.6%;   N: 10.03%;   Co: 20.90%
berechnet   C: 64.30%;   H: 4.68%;   N: 10.00%;   Co: 21.03%

Massenspektrum:
m/e:280 (M$^+$·, 100%); 215 (89%); 189 (10%); 162 (10%); 156 (29%); 140 (14%); 130 (14%); 59 (20%)

**Patentansprüche**

1. Verfahren zur katalytischen, selektiven Herstellung von 2-Vinylpyridin aus Acetylen und Acrylnitril mit Hilfe von Cyclopentadienyl-Cobalt-Katalysatoren bzw. π-Allyl-Cobalt-Katalysatoren bei erhöhter Temperatur unter Verwendung einer mit Acetylen gesättigten Lösung des Nitrils in inerten Lösungsmitteln, dadurch gekennzeichnet, dass die Acrylnitrilkonzentration bis zu 2 Mol/l beträgt und die Umsetzung im Temperaturbereich von 140–180 °C während einer Reaktionsdauer von höchstens 50 Minuten durchgeführt wird.

2. Verfahren nach Anspruch 1, dadurch gekennzeichnet, dass die Temperatur im Bereich von 150 bis 160 °C liegt.

3. Verfahren nach Anspruch 1 und 2, dadurch gekennzeichnet, dass die Reaktionsdauer bei 15 bis 30 Minuten liegt.

4. Verfahren nach Anspruch 1 bis 3, dadurch gekennzeichnet, dass die Acrylnitrilkonzentration um 1 Mol/l liegt.

5. Verfahren nach Anspruch 1 bis 4, dadurch gekennzeichnet, dass man Cobaltocen als Katalysator benutzt.

6. Verfahren nach Anspruch 1 bis 5, dadurch gekennzeichnet, dass man den Katalysator in situ bereitet.

**Revendications**

1. Procédé pour l'obtention catalytique sélective de la 2-vinylpyridine à partir de l'acétylène et du nitrile acrylique à l'aide de catalyseurs de cyclopentadiényl-cobalt ou de catalyseurs de π-allyl-cobalt à température élevée, en utilisant une solution du nitrile dans des solvants inertes saturée d'acétylène, caractérisé en ce que la concentration en nitrile acrylique atteint 2 moles/litre et la réaction est conduite dans une gamme de température de 140 à 180 °C, avec une durée de réaction de 50 minutes au maximum.

2. Procédé selon la revendication 1, caractérisé en ce que la température se situe dans une gamme de 150 à 160 °C.

3. Procédé selon l'une des revendications 1 ou 2, caractérisé en ce que la durée de la réaction est comprise entre 15 et 30 minutes.

4. Procédé selon l'une quelconque des revendications 1 à 3, caractérisé en ce que la concentration en nitrile acrylique atteint 1 mole/litre.

5. Procédé selon l'une quelconque des revendications 1 à 4, caractérisé en ce qu'on utilise le cobaltocène comme catalyseur.

6. Procédé selon l'une quelconque des revendications 1 à 5, caractérisé en ce qu'on prépare le catalyseur in situ.

**Claims**

1. Process for the catalytic, selective preparation of 2-vinylpyridine from acetylene and acrylonitrile with the aid of cyclopentadienylcobalt catalysts or π-allylcobalt catalysts at an elevated temperature, using an acetylene saturated solution of the nitrile in a inert solvent, characterized in that the concentration of acrylonitrile is up to 2 mol/l and that the reaction is carried out in a temperature range of 140 to 180 °C during a reaction time of at most 50 minutes.

2. Process according to claim 1, characterized in that the temperature is in the range of 150 to 160 °C.

3. Process according to claims 1 and 2, characterized in that the reaction time ranges from 15 to 30 minutes.

4. Process according to claims 1 to 3, characterized in that the concentration of acrylonitrile is about 1 mol/l.

5. Process according to claims 1 to 4, characterized in that cobaltocene is used as catalyst.

6. Process according to claims 1 to 5, characterized in that the catalyst is prepared in situ.